# EUROPÄISCHE PATENTANMELDUNG

(11) **EP 2 548 557 A1**
(43) Veröffentlichungstag der Anmeldung: **23.01.2013**
(21) Anmeldenummer: 12005208.9
(22) Anmeldetag: 16.07.2012
(51) Int. Cl.: A61K 31/475, A61P 25/16, A61P 43/00

(54) **Reserpin zur Behandlung von kognitiven Störungen des zentralen Nervensystems**

(30) Priorität: 21.07.2011 DE 102011108116
(71) Anmelder: Dr. Loges + Co. GmbH, 21423 Winsen, Luhe (DE)
(72) Erfinder: Biller, Andreas, 21435 Stelle/ Ashausen (DE); Dimpfel, Wilfried, 35578 Wetzlar (DE)
(74) Vertreter: Henrion, Oliver

(57) **Zusammenfassung**

Die vorliegende Erfindung betrifft Reserpin, Reserpinderivate und/oder deren pharmazeutisch annehmbare Salze oder Komplexe als Agonist des glutamatergen Systems, insbesondere als Agonist der metabotropen Glutamatrezeptoren, bevorzugt als Agonist der metabotropen Gruppe II-Glutamatrezeptoren. Die vorliegende Erfindung betrifft auch Reserpin, Reserpinderivate und/oder deren pharmazeutisch annehmbare Salze oder Komplexe zur Behandlung von kognitiven Störungen des zentralen Nervensystems, insbesondere bei Morbus Parkinson, Demenzen verschiedener Genese, sowie beim Aufmerksamkeits-Defizit-Hyperaktivitäts-Syndrom (ADHS) Die vorliegende betrifft auch ein Arzneimittel zur Behandlung von kognitiven Störungen des zentralen Nervensystems, insbesondere bei Morbus Parkinson, Demenzen verschiedener Genese, sowie beim Aufmerksamkeits-Defizit-Hyperaktivitäts-Syndrom (ADHS), enthaltend Reserpin, dessen Derivate und/oder deren pharmazeutisch annehmbare Salze oder Komplexe und ggf. übliche Hilfs- und Zusatzstoffe.

## Beschreibung

Die vorliegende Erfindung betrifft Reserpin, Resepinderivate und/oder deren pharmazeutisch annehmbare Salze oder Komplexe als Agonist des glutamatergen Systems, insbesondere als Agonist der metabotropen Glutamatrezeptoren, bevorzugt als Agonist der metabotropen Gruppe II-Glutamatrezeptoren. Die vorliegende Erfindung betrifft auch Reserpin, Reserpinderivate und/oder deren pharmazeutisch annehmbare Salze oder Komplexe zur Behandlung von kognitiven Störungen des zentralen Nervensystems, insbesondere bei Morbus Parkinson, Demenzen verschiedener Genese, sowie beim Aufmerksamkeits-Defizit-Hyperaktivitäts-Syndrom (ADHS) und die Verwendung von Reserpin, Reserpinderivate und/oder deren pharmazeutisch annehmbare Salze oder Komplexe zur Behandlung von kognitiven Störungen des zentralen Nervensystems, insbesondere bei Morbus Parkinson, Demenzen verschiedener Genese, sowie beim Aufmerksamkeits-Defizit-Hyperaktivitäts-Syndrom (ADHS). Die vorliegende Erfindung betrifft auch ein Arzneimittel zur Behandlung von kognitiven Störungen des zentralen Nervensystems, insbesondere bei Morbus Parkinson, Demenzen verschiedener Genese, sowie beim Aufmerksamkeits-Defizit-Hyperaktivitäts-Syndrom (ADHS), enthaltend Reserpin, dessen Derivate und/oder deren pharmazeutisch annehmbare Salze oder Komplexe und ggf. übliche Hilfs- und Zusatzstoffe.

Reserpin ist ein Indolalkaloid natürlichen Ursprungs. Der IUPAC-Name von Reserpin ist (1S,2R,3R,4aS,13bR,14aS)-2,11-Dimethoxy-3-(3,4,5-trimethoxybenzoyloxy)-1,2,3,4,4a,5,7,8,13,13b,14,14a-dodecahydroindolo[2',3':3,4]pyrido[1,2-b]isochinolin-1-carbonsäuremethylester (CAS-Nummer 50-55-5) entsprechend der Summenformel C₃₃H₄₀N₂O₉ und der allgemeinen Strukturformel:

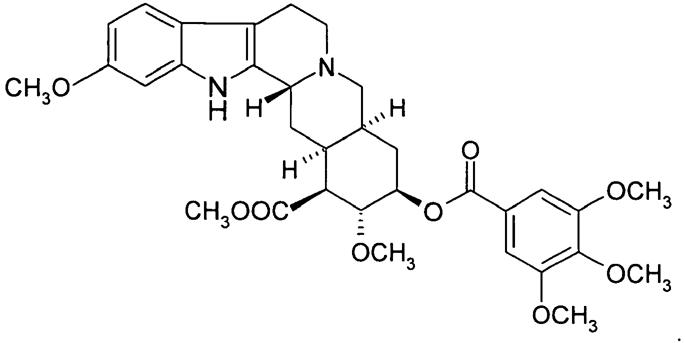

Reserpinderivate sind u.a. beschrieben in der DE 2 418 805 auf die hiermit vollinhaltlich Bezug genommen wird. Reserpin-Methonitrat, beschrieben in S. Sreemantula et al., BMC Pharmacology 2004, 4:30, unter http://biomedcentral.com/1471-2210-4-30, oder Methoxyphenoserpine (Mefeserpine, CAS 3735-85-1) sind weitere beispielhafte Vertreter von ReserpinDerivaten. Reserpinderivate im Sinne der vorliegenden Erfindung weisen eine zu Reserpin analoge biologische oder pharmakologische Wirkung auf.

Reserpin oder dessen Derivate können erfindungsgemäß auch in Form pharmazeutisch annehmbarer Salze oder Komplexe eingesetzt werden. Die Herstellung solcher Salze erfolgt in an sich bekannter Weise. Als Salzbildner kommen alle üblichen pharmazeutisch annehmbaren Säuren bzw. Anionen in Frage, wie HCl, HBr, HI, Sulfate, Phosphate, Formiate, Acetate, Oxalate, p-Tuluolsulfonate, Methylsulfonate, Benzoate und dgl. Umfaßt sind ebenfalls Metabolite und Prodrugs von Reserpin.

Reserpin wurde ursprünglich aus einem Naturprodukt isoliert, insbesondere aus Pflanzen der Gruppe der Schlangenwurzel wie Rauwolfia Serpentina. Reserpin kann jedoch auch synthetisiert werden, wobei zahlreiche Totalsynthesen bekannt sind, wie z.B. von G. Stork (s. D.F. Taber, Org. Chem. Highlights, 2006, May 1, unter http://www.organic-chemistry.org/Highlights/2006/01 May.shtm).

Eine biologische oder pharmakologische Wirkung von Reserpin als Neuroleptikum zur Behandlung der Schizophrenie und als Antihypertonikum zur Behandlung von Bluthochdruck ist bekannt. Es wurden Dosierungen von bis zu 5 mg pro Tag bei der Anwendung als Antihypertonikum und bis zu mehr als 20 mg am Tag bei der Anwendung als Neuroleptikum verabreicht. Heute übliche Dosen bei der Behandlung von Bluthochdruck liegen im Bereich von 0,05 mg bis 0,25 mg am Tag. Kombinationen mit anderen Wirkstoffen, beispielsweise mit Diuretika sind ebenfalls bekannt.

In Tierversuchen wird Reserpin in hoher Dosierung zur Auslösung von Symptomen benutzt, die als Tiermodell einer Depression mit Einschränkung kognitiver Funktionen gelten.

Der Wirkmechanismus von Reserpin wird üblicherweise mit einer Wirkung auf die Konzentration biogener Amine (Noradrenalin, Dopamin und Serotonin) bzw. auf die Monoaminoxidase-Aktivität (MAO) beschrieben. Üblicherweise wird diesbezüglich eine Verarmung an bzw. Entleerung von den genannten (Neuro)Transmittern angenommen.

Die derzeit üblichen Arzneimittel zur Behandlung von kognitiven Störungen des zentralen Nervensystems, insbesondere von Demenzen, Morbus Parkinson, sowie ADHS haben ein breites Nebenwirkungsspektrum. Insbesondere die Behandlung des ADHS wird mit Methylphenidat durchgeführt, obwohl dieses Medikament (Handelsname: Ritalin) eine Amphetamin-ähnliche Wirkung besitzt. Es besteht daher ein großer Bedarf nach einem verbesserten Arzneimittel mit einer guten therapeutischen Wirksamkeit bei möglichst geringer Nebenwirkungsrate. Diese geringe Nebenwirkungsrate ist bei Verabreichung eines Naturstoffes, insbesondere in niedriger Dosierung, eher zu erwarten.

Überraschend wurde nunmehr gefunden, daß Reserpin eine biologische oder pharmazeutische Wirkung in Bezug auf kognitive Hirnfunktionen aufweist und zwar im Sinne einer aktivierenden Substanz. Besonders überraschend zeigt sich die Wirkung bereits bei niedrigen und ultra-niedrigen Dosierungen. Die Anwendung der völlig "bias"-freien Untersuchung im Tele-Stereo-EEG der Ratte erbrachte im Rahmen von Versuchen mit anderer Zielsetzung den Hinweis auf das Vorhandensein einer derartigen aktivierenden Wirkung im Bereich kognitiver Hirnfunktionen. Die überraschend gefundene biologische oder pharmazeutische Wirkung bzw. Aktiviät wurde in vivo an der Ratte und in vitro dokumentiert.

Es wird angenommen, daß die vorgenannte Aktiviät auf einer Wirkung von Reserpin auf das glutamaterge System, speziell auf metabotrope Glutamatrezeptoren beruht.

Die Erfindung betrifft daher Reserpin, Resepinderivate und/oder deren pharmazeutisch annehmbare Salze oder Komplexe zur Behandlung von kognitiven Störungen des zentralen Nervensystems, insbesondere bei degenerativen Erkrankungen, wie Demenzen, Morbus Parkinson und dem Aufmerksamkeits-Defizit-Hyperaktivitäts-Syndrom (ADHS).

Im Rahmen der vorliegenden Erfindung sollen, sofern nicht anders angegeben, von dem Begriff "Reserpin" alle vorgenannten Erscheinungsformen, d.h. Reserpin, dessen Derivate und pharmazeutisch annehmbare Salze oder Komplexe derselben umfaßt sein. Besonders bevorzugt wird Reserpin als freie Base und/oder in Form seiner pharmazeutisch annehmbaren Salze eingesetzt.

Unter kognitiven Störungen sollen vorliegend insbesondere Aufmerksamkeits- und Konzentrationsstörungen, Gedächtnisstörungen, Sprachstörungen, Orientierungsstörungen, Störungen der motorischen Kontrolle, wie Probleme der motorischen Reaktionsgeschwingigkeit, und Störungen der Problemlösefähigkeit verstanden werden.

Unter üblichen Dosierungen werden vorliegend Dosen im Bereich von ca. 0,05 mg bis ca. 0,25 mg am Tag verstanden. Niedrige Dosierungen betreffen Dosen kleiner ca. 0,05 mg am Tag bis ca. 0,1 µg am Tag, beispielsweise kleiner ca. 0,03 mg am Tag, wie ca. 0,002 mg - ca. 0,02 mg am Tag, und ca. 0,006 - 0,01 mg am Tag. Unter ultra-niedrigen Dosierungen sollen Dosen kleiner ca. 0,1 µg am Tag verstanden werden, z.B. ca. 0,001 bis ca. 0,06 µg pro Tag. Innerhalb der genannten Werte können geeignete, auch übergreifende, Bereiche ausgwählt werden, wie z.B. ca. 0,001 µg bis ca. 0,03 mg.

In in vitro Untersuchungen konnte eine Rezeptor-vermittelte Wirkung von Reserpin nachgewiesen werden. Diese Wirkung betrifft vorzugsweise die metabotropen Glutamatrezeptoren, insbesondere die metabotropen Gruppe II Glutamatrezeptoren.

Metabotrope Glutamatrezeptoren sind im Unterschied zu den ebenfalls im glutamatergen System enthaltenden ionotropen Rezeptoren keine einen Kanal steuernde Rezeptoren, sondern G-Protein-gekoppelte Rezeptoren. Die metabotropen Glutamatrezeptoren der Gruppe II, d.h. der mGluR2- und der mGluR3-Rezeptor, hemmen beispielsweise die Adenylatcyclase. Insofern wird vorliegend ein neuer, von der bekannten Wirkung auf Monoaminoxidase-Aktivität (MAO) verschiedener Wirkmechanismus beschrieben.

Die vorliegende Erfindung betrifft daher auch die Eigenschaften von Reserpin, Resepinderivaten und/oder deren pharmazeutisch annehmbaren Salzen oder Komplexen als Agonist des glutamatergen Systems, insbesondere der metabotropen Glutamatrezeptoren, bevorzugt als Agonist der metabotropen Gruppe II-Glutamatrezeptoren. Entsprechend ist ein weiterer Gegenstand der vorliegenden Erfindung Reserpin, Reserpinderivate und/oder deren pharmazeutisch annehmbare Salze oder Komplexe zur Behandlung von Zuständen, die durch eine Hemmung des glutamatergen Systems, insbesondere der metabotropen Glutamatrezeptoren, bevorzugt durch eine Hemmung der metabotropen Gruppe II-Glutamatrezeptoren bedingt sind. Derartige Zustände sind beispielsweise kognitive Störungen und Beeinflussungen der Modulation synaptischer Übertragung und der Induktion synaptischer Plasizität, wie (Langzeit-)Potentierung und (Langzeit-)Depression der synaptischen Signalübertragung.

Die vorliegende Erfindung betrifft auch die Verwendung von Reserpin, Reserpinderivaten und/oder deren pharmazeutisch annehmbaren Salzen oder Komplexen zur Behandlung von kognitiven Störungen des zentralen Nervensystems, insbesondere bei degenerativen Erkrankungen, wie Demenzen, Morbus Parkinson und dem Aufmerksamkeits-Defizit-Hypearaktivitäts-Syndrom (ADHS). Die Erfindung betrifft auch die Verwendung von Reserpin, Resepinderivaten und/oder deren pharmazeutisch annehmbaren Salzen oder Komplexen als Mittel zur Behandlung von kognitiven Störungen des zentralen Nervensystems, insbesondere bei degenerativen Erkrankungen, wie Demenzen, Morbus Parkinson und dem Aufmerksamkeits-Defizit-Hyperaktivitäts-Syndrom (ADHS), sowie deren Verwendung zur Herstellung entsprechender Mittel. In diesem Zusammenhang ist auch die Herrichtung von Reserpin, Reserpinderivaten und/oder deren pharmazeutisch annehmbaren Salzen oder Komplexen als ein entsprechendes Mittel mit umfaßt.

Weiterer Gegenstand der vorliegenden Erfindung ist ein Mittel enthaltend Reserpin, Reserpinderivate und/oder deren pharmazeutisch annehmbare Salze oder Komplexe zur Behandlung von kognitiven Störungen des zentralen Nervensystems, insbesondere bei degenerativen Erkrankungen, wie Demenzen, Morbus Parkinson und dem Aufmerksamkeits-Defizit-Hypearaktivitäts-Syndrom (ADHS). Bevorzugt enthält das Mittel einen therapeutisch wirksamen Gehalt an dem Wirkstoff. Optional enthält das Mittel übliche Hilfs- und Zusatzstoffe.

Zusätzlicher Gegenstand der Erfindung ist ein Verfahren zur Behandlung von kognitiven Störungen des zentralen Nervensystems, insbesondere bei degenerativen Erkrankungen, wie Demenzen, Morbus Parkinson und dem Aufmerksamkeits-Defizit-Hyperaktivitäts-Syndrom (ADHS) umfassend die Gabe Reserpin, Reserpinderivaten und/oder deren pharmazeutisch annehmbaren Salzen oder Komplexen oder eines erfindungsgemäßen Mittels an einen Patienten, der eine solche Behandlung benötigt in einer therapeutisch wirksamen Menge.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, daß zumindest um 10 % höhere oder niedrigere Werte oder um 5 % höhere oder niedrigere Werte und in jedem Fall um 1 % höhere oder niedrigere Werte eingeschlossen sind. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die erfindungsgemäßen Reserpin als Wirkstoff enthaltenden (Arznei)Mittel werden bevorzugt oral verabreicht. Es ist jedoch auch eine Verabreichung auf anderem Wege, z.B. peroral, topisch, parenteral, intravenös, intramuskulär, subkutan, nasal, inhalativ oder transdermal möglich.

Zur Verabreichung kann das erfindungsgemäße Mittel z.B. in Form von Tabletten, Kapseln, Pillen, Dragees, Granulaten, Suppositorien, Pellets, Lösungen, Dispersionen formuliert werden, wobei optional pharmazeutisch annehmbare Hilfs- und Trägerstoffe enthalten sind.

Liegt das erfindungsgemäße Mittel in oraler Darreichungsform z.B. als eine Lösung vor, so enthält diese typischerweise 0,01 - 0,05 µg /ml, bevorzugt 0,03 µg/ml des Wirkstoffs Reserpin. Andere Gehalte sind möglich und können vom Fachmann mittels einfacher Versuche, ggf. unter Berücksichtigung des Alters, Gewichts, Geschlechts und weiterer Parameter eines konkreten Patienten ermittelt werden.

Die erfindungsgemäßen Mittel werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silikat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinylpyrrolidon: Quell- und Sprengmittel, z.B. Stärke, Alginsäure, Alginate oder Natriumstärkeglykolate, aufschäumende Mischungen; Farbstoffe; Süßungsmittel; Benetzungsmittel, wie Lecithin, Polysorbate, Laurylsulfate; und im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Flüssige Darreichungsformen zur oralen Verabreichung können z.B. Sirupe, Emulsionen und Suspensionen oder Dispersionen sein.

Ein Sirup kann als Träger z.B. Saccharose oder Saccharose mit Glycerin und/oder Mannitol und/oder Sorbitol enthalten.

Suspensionen und Emulsionen können als Träger z.B. ein natürliches Harz, Agar, Natriumalginat, Pectin, Methylcellulose, Carboxymethylcellulose oder Polyvinylalkohol enthalten.

Suspensionen oder Lösungen für intramuskuläre Injektionen können zusammen mit dem Wirkstoff einen pharmazeutisch annehmbaren Träger, z.B. steriles Wasser, Olivenöl, Ehyloleat, Glykole, z.B. Propylenglykol, und, erforderlichenfalls, eine geeignete Menge an Lidocain-Hydrochlorid enthalten.

Lösungen zur intravenösen Injektion oder Infusion können als Träger z.B. steriles Wasser enthalten oder sie können bevorzugt in Form von sterilen, wässrigen, isotonischen Salzlösungen vorliegen.

Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Eine Dosierungseinheit des Mittels kann beispielsweise enthalten:
bei peroralen Arzneiformen:
   0,001 µg - 0,03 mg, bevorzugt 0,01 bis 0,06 µg, besonders bevorzugt 0,03 bis 0,04 µg Wirkstoff pro Tagesdosis.
      Die Tagesdosis kann beispielsweise in 1 bis 3 Einzeldosen, vorzugsweise in zwei Einzeldosen, täglich verabreicht werden.
bei parenteralen Arzneiformen (zum Beispiel intravenös, subkutan, intramuskulär):
   0,001 µg - 0,03 mg, bevorzugt 0,01 bis 0,05 µg, besonders bevorzugt 0,03 µg Wirkstoff pro Tagesdosis.
      Die Tagesdosis kann beispielsweise in 1 bis 3 Einzeldosen, vorzugsweise in einer Einzeldosis, täglich verabreicht werden.

Eine Anpassung der konkreten Dosis in Abhängigkeit vom Alter, Geschlecht, Gewicht, Zustand, etc. des Patienten und des verwendeten Wirkstoffs (als reine Verbindung, Salz, Komplex, etc.) liegt im Können des Fachmanns und erfolgt auf dem Fachmann bekannte Weise.

Nachfolgend wird eine kurze Beschreibung der Abbildungen gegeben.

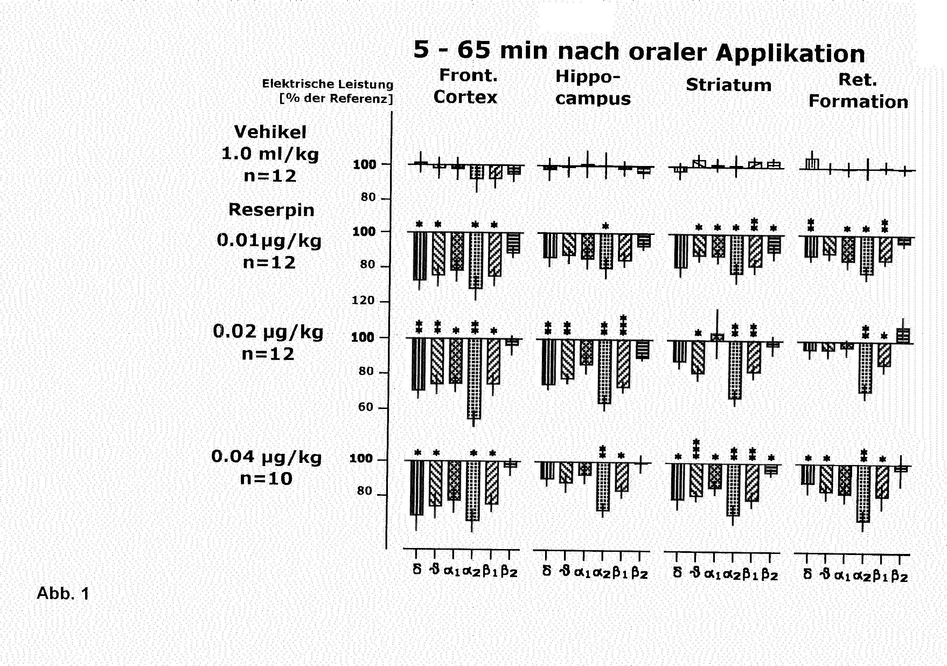

Abb. 1 zeigt eine Dosis-abhängige Wirkung von Reserpin in vivo im Vergleich zur Applikation von Vehikel (Dosis: 0,01 µg/kg, 0,02 µg/kg und 0,04 µg/kg). Auf der y-Achse ist die Dosis aufgetragen. Die x-Achse gibt die Frequenzbereiche nach der Fast Fourier Transformation der Daten an: delta (δ), theta (θ), alpha1 (α1), alpha2 (α2), beta1 (β1) und beta2 (β2). Mittelwerte von n=12 Tieren. Sterne kennzeichnen die statistische Signifikanz, berechnet nach Wilcoxon, Mann und Whitney, zweiseitig: *=p<0.05; **=p<0.01; ***=p<0.001.

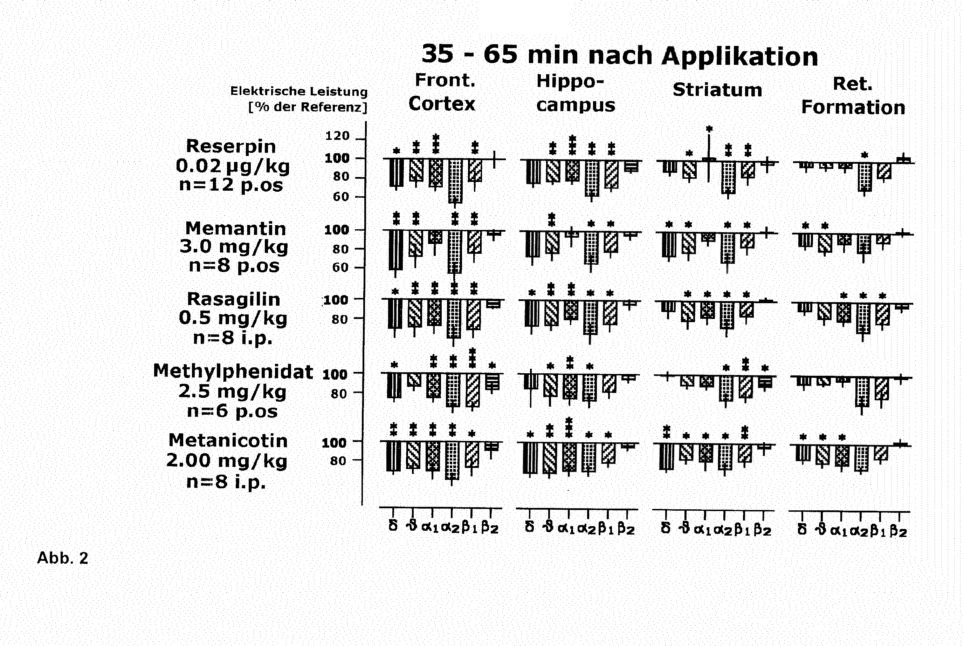

Abb. 2 zeigt die Wirkung von 0,020 µg/kg Reserpin auf EEG Frequenzen im Vergleich zu Standardmedikamenten. Mittelwerte von n=7-12 Tieren im jeweiligen Experiment. Weitere Details s. Abb. 1.

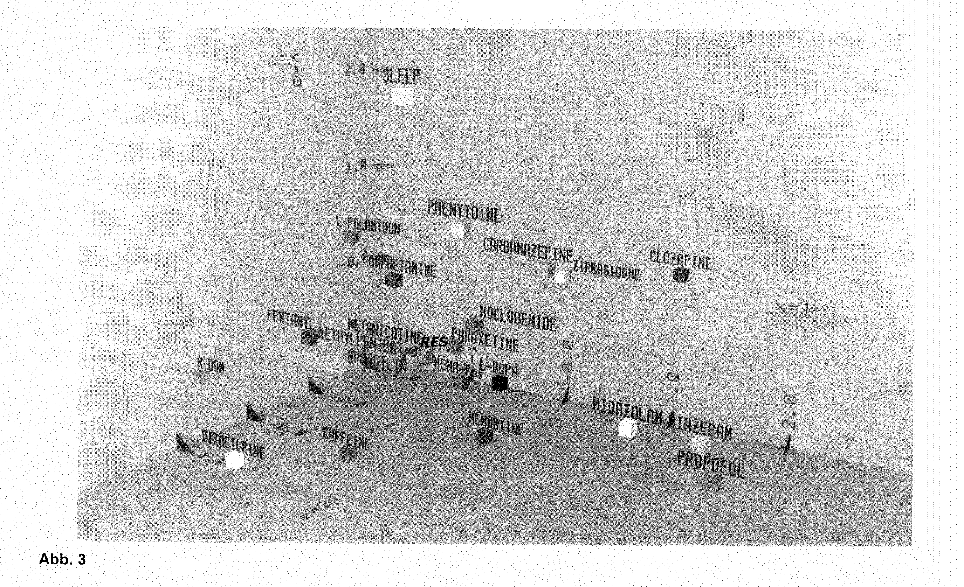

Abb. 3 die Dokumentation des Vergleiches der Wirkung von Reserpin mit Referenzmedikamenten anhand der Ergebnisse einer linearen Diskriminanzanalyse auf der Basis von vier Hirnregionen und 6 Frequenzbändern (24 Variablen). RES steht für Reserpin, Referenzmedikamente sind mit vollem Namen angegeben. Die Ergebnisser der ersten und zweiten Diskriminanzfunktion sind auf der x- und y-Achse dargestellt. Das Ergebnis der dritten Diskriminanzfunktion befindet sich auf der z-Achse. Die Ergebnisse der vierten bis sechsten Diskriminanzfunktionen sind anhand einer Farbmischung dargestellt, sind jedoch in dieser nicht farbigen Darstellung nicht sichtbar. Die sich ergebenden Farben (hier vorliegend in Graustufen dargestellt) entstehen aus einer additiven Farbmischung aus den Farben rot-grün-blau stellvertretend für die Ergebnisse der vierten, fünften und sechsten Diskriminanuzfunktion. Die Wirkung von Reserpin ordnet sich in die Nachbarschaft von Metanikotin (stabiles Nikotinderivat), Memantin, Methylphenidat und Rasagilin ein (zum Teil auch ähnliche Farbe, d.h. in dieser Darstellung als Nachbarn mit ähnlicher Graustufe).

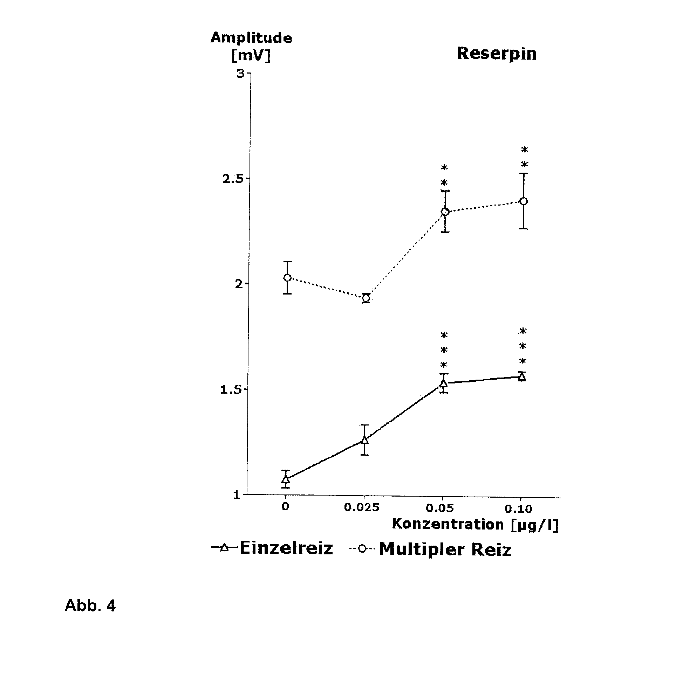

Abb. 4 zeigt die Wirkung von Reserpin im Hippokampus-Schnittpräparat in vitro. Die Erfassung des Populationsspikes der Pyramidenzellen erfolgt nach elektrischer Reizung der Schaffer-Kollateralen in Form von Einzelimpulsen sowie in Gegenwart multipler Reize zur Darstellung der Langzeitpotenzierung. Sterne kennzeichnen die statistische Signifikanz, berechnet nach Wilcoxon, Mann und Whitney, zweiseitig: *=p<0.10; **=p<0.05; ***=p<0.01.

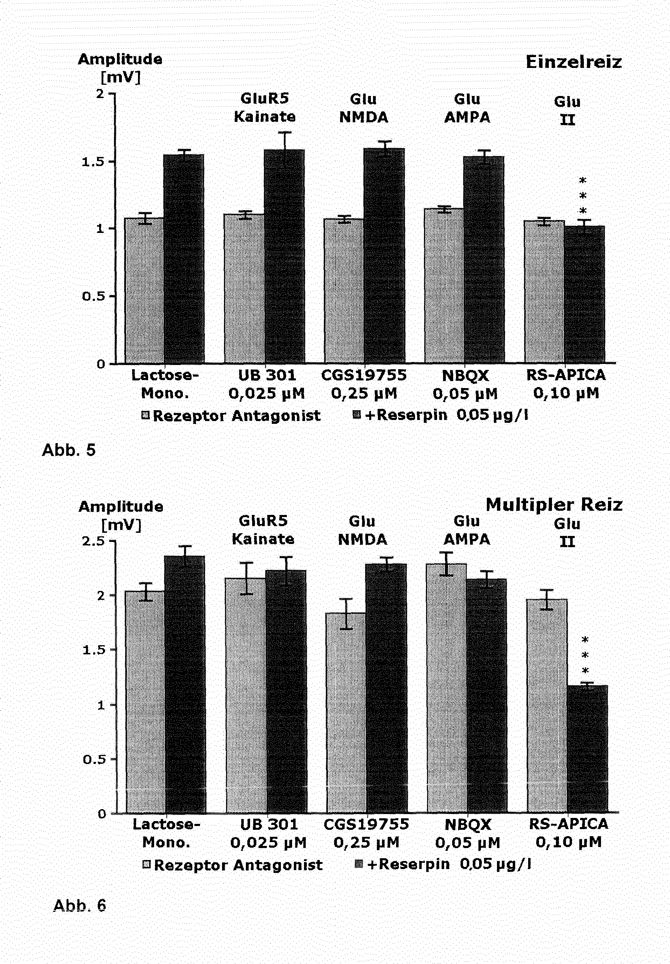

Abb. 5 zeigt die Wirkung von Reserpin im Hippokampus-Schnittpräparat in vitro. Die Erfassung des Populationsspikes der Pyramidenzellen erfolgt in Gegenwart verschiedener Rezeptor Antagonisten des Neurotransmitters Glutamat bei Reizung mit einzelnen elektrischen Impulsen. Die Antagonisten wurden mit der üblichen Kurzform auf der x-Achse bezeichnet.

Abb. 6 zeigt die Wirkung von Reserpin im Hippokampus-Schnittpräparat in vitro. Die Erfassung des Populationsspikes der Pyramidenzellen erfolgt in Gegenwart verschiedener Rezeptor Antagonisten des Neurotransmitters Glutamat bei Reizung mit multiplen elektrischen Impulsen. Die Antagonisten wurden mit der üblichen Kurzform auf der x-Achse bezeichnet.

### Beispiel 1 (Tele-Stereo-EEG)

Die Veränderungen der EEG-Frequenzen wurde nach oraler Gabe von Lactosemonohydrat (Kontrolle/Vehikel) bzw. Verdünnungen von Reserpin in Lactosemonohydrat entsprechend 0,01, 0,02 oder 0,04 µg Reserpin/kg Körpergewicht) bestimmt.

Die Untersuchungen wurden analog der durch W. Dimpfel beschriebenen Methode (Dimpfel W Preclinical data base of pharmaco-specific rat EEG fingerprints (Tele-Stereo-EEG). Eur J Med Res (2003) 8: 199-207; auf dieses Dokument wird vollinhaltlich bezug genommen) folgendermaßen durchgeführt:

Zwölf männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-FourierTransformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen: die Substanzen wurden oral 45 Minuten nach Beginn der Messungen (Vorwert) appliziert. Fünf Minuten später wurden die Messungen wieder gestartet, mindestens über die nächsten 5 Stunden kontinuierlich analysiert und in 60-minütigen Perioden zusammengefasst. Die Testsubstanz wurde in einer Dosierung von 0,01, 0,02 und 0,04 µg/kg appliziert. Die experimentelle Serie wurde mit der Applikation von Lactosemonohydrat (Kontrolle), die zu keiner auffälligen Änderung führte, begonnen (oberste Zeile in Abb. 1).

Der statistische Vergleich der Versuche zu den Ergebnissen, die nach Gabe des Vehikels gemessen wurde, erfolgte mit Hilfe des statistischen Tests nach Wilcoxon, Mann und Whitney auf der Basis der Veränderungen innerhalb der einzelnen Frequenzbänder in allen Hirnregionen als Variablen.

Die Verabreichung von Lactosemonohydrat führte kaum zu Veränderungen der elektrischen Leistung (µV²/Ω) im Vergleich zu den Vorphasenwerten (Abb. 1).

Die Verabreichung von Reserpin führte zu einer dosis-abhängigen Unterdrückung der spektralen Leistung in allen Frequenzbereichen. Besonders stark waren die alpha2 Wellen betroffen. Eine Steigerung der Motilität der Tiere wurde nicht beobachtet.

Die Untersuchungen der Veränderungen der EEG-Frequenzen der Ratte nach Gabe von Reserpin zeigen überraschenderweise im Vergleich zur Gabe von Vehikel (Trägersubstanz Lactosemonohydrat), dass das Präparat eine Wirkung im Gehirn besitzt, die bei der Behandlung von kognitiven Beeinträchtigungen des Gehirns, insbesondere bei Morbus Parkinson, Demenzen verschiedener Genese, sowie beim Aufmerksamkeits-Defizit-Hyperaktivitäts-Syndrom (ADHS) nützlich ist (Abb.1). Die Wirkung von Reserpin wird bereits in ultra-niedrigen Konzentrationen erreicht (Abb. 1).

Die Abb. 2 zeigt die Veränderungen der EEG-Frequenzen nach Gabe einer ultra-niedrigen Einzel-Dosis von Reserpin im Vergleich zu verschiedenen Medikamenten mit bekannter klinischer Indikation. Reserpin zeigt überraschenderweise im Modell Tele-Stereo-EEG bei Ratten dosisabhängige Veränderungen der EEG-Frequenzen, wie sie nach Gabe klassischer Medikamente zur Behandlung Demenzen (Beispiel Memantin), Morbus Parkinson (Beispiel Rasagilin), wie auch ADHS (Beispiel Methylphenidat) gemessen werden. Die Veränderungen, wie sie nach Gabe von einer Dosis von Reserpin (0,02 und 0,04 µg/kg) auftreten, sowie nach Gabe klassischer Medikamente zur Behandlung degenerativer Krankheiten mit Beeinträchtigung kognitiver Funktionen verwendet werden (Abb. 2) zeigen eine große Ähnlichkeit untereinander. Die in vivo Versuche an der Ratte zeigen somit für Reserpin Ergebnisse, wie sie für Medikamente zur Behandlung von Demenzen, Morbus Parkinson sowie ADHS typisch sind (Abb. 2). Auch die Ähnlichkeit zur Wirkung des stabilen Nikotinderivates Metanicotin läßt auf eine mentale Leistungssteigerung schließen.

Die beobachteten Frequenzänderungen wurden mittels einer Diskriminanzanalyse mit den Ergebnissen von Medikamenten zur Behandlungvon degenerativen Erkrankungen des Gehirns wie auch mit Medikamenten für andere Indikationen verglichen. Arzneimittel, welche bereits im selben Modell unter identischen Bedingungen geprüft wurden, standen für Vergleichszwecke zur Verfügung (siehe nachfolgendeTabelle). Die Angaben dort beziehen sich jeweils auf eine Dosierung in mg/kg Körpergewicht.

**Tabelle: Auflistung der in der Diskriminanzanalyse verwendeten Referenzsubstanzen. Die unter Definition gelisteten Substanzen wurden für die Erstellung der Diskriminanzfunktionen verwendet. Die unter Analyse aufgelisteten Substanzen wurden unter Verwendung dieser Funktionen unter Verwendung derselben Projektionsparameter abgebildet.**

| **Substanze Definition** | **Dosis [mg/kg)** | **Zeit** | **Substanze Analyse** | **Dosis [mg/kg]** | **Zeit** |
|---|---|---|---|---|---|
| Coffein | 2.50 | 35 - 65 min | Metanicotin | 2.00 | 35 - 65 min |
| Diazepam | 0.50 | 35 - 65 min | Midazolam | 0.50 | 35 - 65 min |
| Dizoclipin | 0.25 | 35 - 65 min | R-DOM | 0.20 | 35 - 65 min |
| Moclobemid | 5.00 | 35 - 65 min | Paroxetin | 2.00 | 35 - 65 min |
| L-Polamidon | 1.00 | 35 - 65 min | Fentanyl | 0.075 | 35 - 65 min |
| Ziprasidone | 1.00 | 35 - 65 min | Cbzapin | 3.00 | 35 - 65 min |
| (+)Amphetamin | 0.20 | 35 - 65 min | L-Dopa | 2.50 | 35 - 65 min |
| Propofol | 40.0 | 35 - 65 min | Schlaf | | 35 - 65 min |
| Memantin i.p. | 3.00 | 35 - 65 min | Memantin p.os | 5.00 | 35 - 65 min |
| Phenytoin | 4.00 | 95 - 125 min | Carbamacepin | 15.0 | 95 - 125 min |
| | | | Methylphenidat | 2.50 | 35 - 65 min |
| | | | Rasagilin | 0.50 | 35 - 65 min |
| | | | Reserpin | 0.02 [µg/kg] | 35 - 65 min |

Das aufgefundene Frequenzmuster für Reserpin korreliert in auffälliger Weise mit den Mustern, die für Medikamente, wie sie bereits für die Behandlung von Demenz (Memantin, Morbus Parkinson (Rasagiline) sowie ADHS (Methylphenidat) üblicherweise verwendet werden, nicht jedoch mit Mustern, die bei Medikamenten für andere Indikationen auftreten (Abb. 3).

Die Diskriminanzanalyse hat gezeigt, dass sich der charakteristische elektrische Fingerprint, der für klassische Medikamente zur Behandlung von Demenz, Morbus Parkinson, sowie ADHS typisch ist, auch in den Mustern von Reserpin in auffälliger Weise wiederfindet. Aus der Erkenntnis, dass Reserpin die gleichen charakteristischen Veränderungen der EEG-Frequenzen hervorruft wie üblicherweise Arzneimittel, die zur Behandlung von Demenz, Morbus Parkinson, und ADHS eingesetzt werden, kann gefolgert werden, dass Reserpin bei der Behandlung in denselben klinischen Indikationen wirksam ist. Dass Medikamente, die in der gleichen Indikation eingesetzt werden, auch gleichartige EEG Veränderungen hervorrufen, konnte von Dimpfel anhand von mehr als 40 Referenzsubstanzen für 8 verschiedene Indikationen gezeigt werden (Dimpfel W Preclinical data base of pharmaco-specific rat EEG fingerprints (Tele-Stereo-EEG). Eur J Med Res (2003) 8: 199-207).

### Beispiel 2 (Langzeitpotenzierung im Hippokampus-Schnittpräparat in vitro)

Wirkungen von Reserpin auf die Erregbarkeit der Pyramidenzellen wurden im Hippokampus-Schnittpräparat der Ratte bestimmt. Die Analyse der Wirkungen erfolgte auf zwei Ebenen. Zunächst wurde anhand der Applikation von Einzelreizen die normale Erregbarkeit überprüft und anschließend nach einer sog. "Theta-burst-Stimulation" die Auswirkungen auf die Langzeitpotenzierung gemessen. Im nächsten Schritt wurde versucht, die Wirkung von Reserpin mit Hilfe von verschiedenen Rezeptor-spezifischen Glutamat Antagonisten zu unterdrücken.

Für die Durchführung dieser Studien wurden 9 erwachsene männliche CD-Ratten verwendet. Die Isolation des Hippokampus erfolgte an äthernarkotisierten und anschließend exsanguinierten Tieren. In Phosphatgepufferter Salzlösung (NaCl: 124 mM; KCI: 5 mM; CaCl: 2 mM; MgSO4: 2 mM; NaH2PO4: 1,25 mM; NaHCO3: 26 mM: Glucose: 10 mM; Kontroll-Lösung: Artifizielle Cerebral-Spinal-FLüsssigkeit (ACSF); alle Substanzen stammen von der Firma Roth, Karlsruhe, DE. Dann wurde der mittlere Teil des Hippokampus mit Hilfe eines Schnellklebers aufgeblockt und mit einem Vibratom in 400 µm dicke Scheiben geschnitten. Die Aufbewahrung dieser Schnitte erfolgte für mindestens eine Stunde vor Versuchsbeginn (siehe S.J. Schiff, G.G. Somjen "The effects of temperature on synaptic transmission in hippocampal tissue slices" Brain Research (1985), 345: 279-284) in einer mit Carbogen durchperlten Inkubationskammer.

Das Experiment selbst wurde in einer sog. "Base Unit" mit Haas Top" (Firma Medical Systems Corp., USA) bei 35 Grad Celsius durchgeführt. Der mit Hilfe peristaltischer Pumpen (Infusomat B. Braun Melsungen AG) superfundierte Hippokampus-Schnitt lag auf einem Stück Gaze. Eingeleitetes Carbogen hielt die erforderliche Sauerstoffzufuhr der Lösung aufrecht. Die Durchflussgeschwindigkeit betrug 200 ml/h.

Die Stimulation der CA2-Region (Schaffer-Kollateralen) erfolgte unter Verwendung eines Reizgenerators (Laborcomputer Labteam der Fa. MediSyst GmbH, Linden, DE) über eine Isoliereinheit mit Hilfe einer bipolar konzentrischen Stahlelektrode (Rhodes Medical Systems, USA. Die Impulsbreite betrug 200 µs, die Stromstärke konstant 200 pA. Der Reizgenerator löste im Abstand von jeweils 20 Sekunden 4 Einzelreizungen aus, die im Schnitt insgesamt 4 Populationsspikes evozierten. Die Ableitung der Antwort erfolgte in der CA3 Region. Das System mittelte die Reizantwort der 4 Spike-Amplituden.

Die Wirkung von Reserpin auf das evozierte Potential wurde nach Einzelreizung wie auch nach Induktion der Langzeitpotenzierung durch einen kurzdauernden (1 s) tetanischen Reiz (90 Hz) untersucht (siehe K.G. Reymann, H.K. Matthies, U. Frey, V.S. Voborbyev, H. Matthies "Calcium-induced long-term potentiation in the hippocampal slice. Characterization of the time course and conditions"", Brain Bulletin (1986), 17: 291-296). Dabei wurde der Schnitt zunächst mit Kontrolllösung superfundiert. Nach Auffinden eines geeigneten Signals und Registrierung dieses Ausgangssignals während mehrerer Zeitpunkte wurde anstelle der Kontrolllösung auf Reserpin-haltige Lösung umgeschaltet. Jeder Schnitt wurde jeweils nur zu einem Experiment benutzt. Werte werden für n=4 Schnitte angegeben.

Die Ableitung der evozierten Antwort erfolgte in 10-minütigem Abstand extrazellulär mit einer gezogenen Glaskapillare (Elektrodenpuller, Rhema Labortechnik, DE). Das Antwortsignal wurde verstärkt (Verstärker "LMI", List Electronics, Darmstadt, DE) mit einem Laborrechnersystem Labteam (Software NeuroTool, MediSyst GmbH, Linden DE) analog digital gewandelt (Auflösung 12 Bit) und ausgewertet. Nach Auffinden eines geeigneten Signals (Amplitudenhöhe etwa 1 mV) wurde die Amplitudenhöhe des Populationsspikes (SAP=Summenaktionspotential; Popspike) als Ausgangsgröße festgelegt. Dieser Referenzwert ergab sich aus dem Mittelwert der letzten drei gemessenen Amplitudenhöhen während Superfusion mit der ACSF Lösung (Ausgangswert). Danach erfolgte die Superfusion mit Testsubstanz mit gleichem Procedere. In Folgeexperimenten erfolgte eine kurzfristige tetanische Reizung (Theta-Burst-Stimulus=TBS) zur Induktion der Langzeitpotenzierung. Hieraus resultierende Amplitudenveränderungen wurden in % dieses Ausgangswertes (n=4) angegeben. Werte in Gegenwart der Testsubstanz sind in % Reduktion dieses Referenzwertes angegeben.

Desweiteren wurde die Erregbarkeit der Pyramidenzellen in der Ausgangslage durch Superfusion mit einem Glutamat Rezeptor Antagonisten verändert. In der Folge wurde dann gleichzeitig Reserpin mit den verschiedenen Antagonisten zusammen superfundiert, um eine antagonistische Wirkung zu erkennen.

Die Ergebnisse dieser Untersuchungen sind in den Abb. 4 bis 6 gezeigt.

Die erste Versuchsanordnung zeigt eine Konzentrations-abhängige Zunahme der Amplitude des Populationsspikes in Gegenwart von 0,025-0,1 µg/Liter Reserpin bei Einzelreizung (siehe Abb. 4). Die Auslösung der Langzeitpotenzierung durch TBS wurde Konzentrations-abhängig verbessert (ebenfalls Abb. 4), wie es von Versuchen mit Gedächtnis-steigernden Substanzen wie Memantin her bekannt ist (siehe W. Dimpfel "Effects of memantine on synaptic transmission in the hippocampus in vitro" Arzneimittel-Forschung/Drug Research (1995), 45: 1-5).

Dieselbe Versuchsanordnung wurde noch einmal genutzt, um in Gegenwart verschiedenster Glutamat Rezeptor Antagonisten Hinweise für eine Interaktion zwischen Reserpin und einem der Glutamat-Rezeptoren (GluR5 Kainate steht dabei für den ionotropen GluR5 Kainate-Rezeptor, Glu NMDA - für ionotrope NMDA-Rezeptoren, Glu AMPA - für ionotrope AMPA-Rezeptoren, und Glu II - für metabotrope Gruppe II Rezeptoren) zu erhalten (Abb. 5 und Abb.6).

Es wurden vier Antagonisten getestet. Dabei steht UB 301 für den Kainate-Rezeptor-Antagonisten UBP 301, CAS-Nr. 569371-10-4, der Formel 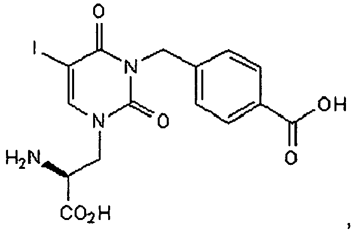 CGS19755 - für den NMDA-Rezeptor-Antagonisten CGS 19755, CAS-Nr. 110347-85-8, der Formel 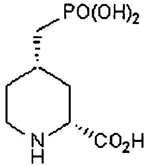 NBQX - für den AMPA-Rezeptor-Antagonisten NBQX, CAS-Nr. 118876-58-7, der Formel 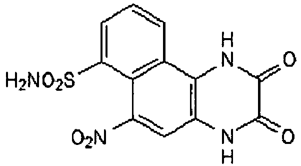 und RS-Apica - für den Antagonisten für metabotrope Gruppe II Rezeptoren (RS)-Apica, (RS)-1-Amino-5-phosphono-indan-1-carbonsäure, der Formel 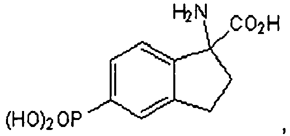 wie sie von der Firma Tocris Bioscience kommerziell ,erhältlich sind (s. unter http://www.tocris.com). Von den getesteten 4 Antagonisten (UB 301, CGS19755, NBQX und RS-Apica) konnte RS-Apica, ein metabotroper Glutamat Gruppe II Rezeptor Antagonist, die Wirkung von Reserpin besonders ausgeprägt unterdrücken. Bei multiplem Reiz ist jedoch auch eine Unterdrückung durch NBQX, einen AMPA-Rezeptor-Antagonisten zu verzeichnen (Abb. 5, Abb.6).

Die Untersuchung der Wirkung von Reserpin im Hippokampus-Schnittpräparat zeigte eine Konzentrations-abhängige Zunahme des Populationsspikes der Pyramidenzellen. Bei Auslösung der Langzeit-Potenzierung (LTP) durch multiple elektrische Reizung wurde eine Steigerung der LTP gemessen, wie sie auch in der Vergangenheit in Gegenwart von Memantin, einem Medikament, das bei Demenzen verschrieben wird, beschrieben wurde (Abb. 6). Eine Zunahme der LTP wird üblicherweise in Relation zu einer Verbesserung des räumlich-zeitlichen Gedächtnisses gesehen. Die Konzentrations-abhängige Wirkung konnte durch Gegenwart eines Antagonisten der metabotropen Gruppe II Glutamatrezeptoren ausgeprägt unterdrückt werden, jedoch auch durch einen Antagonisten der ionogenen AMPA-Rezeptoren. Alle Ergebnisse stehen mit der Vorstellung einer Verbesserung kognitiver Prozesse im Einklang.

Die Versuchsergebnisse demonstrieren somit eine Wirkung von Reserpin auf das glutamaterge System, insbesondere als Agonist metabotroper Glutamatrezeptoren, speziell als Agonist metabotroper Gruppe II-Glutamatrezeptoren.

Die vorgenannten Beispiele dienen der Erläuterung der Erfindung, ohne daß die Errfindung jedoch auf die speziell beschriebenen Ausführungsformen beschränkt sein soll.

## Patentansprüche

1. Reserpin, Reserpinderivate und/oder deren pharmazeutisch annehmbare Salze oder Komplexe als Agonist des glutamatergen Systems.

2. Reserpin, Reserpinderivate und/oder deren pharmazeutisch annehmbare Salze oder Komplexe nach Anspruch 1 als Agonist der metabotropen Glutamatrezeptoren, bevorzugt als Agonist der metabotropen Gruppe II-Glutamatrezeptoren.

3. Reserpin, Reserpinderivate und/oder deren pharmazeutisch annehmbare Salze oder Komplexe zur Behandlung von Zuständen, die durch eine Hemmung des glutamatergen Systems bedingt sind.

4. Reserpin, Reserpinderivate und/oder deren pharmazeutisch annehmbare Salze oder Komplexe nach Anspruch 3, wobei die Zustände durch eine Hemmung der metabotropen Glutamatrezeptoren, insbesondere der metabotropen Gruppe II-Glutamatrezeptoren bedingt sind.

5. Reserpin, Reserpinderivate und/oder deren pharmazeutisch annehmbare Salze oder Komplexe zur Behandlung von kognitiven Störungen des zentralen Nervensystems.

6. Reserpin, Reserpinderivate und/oder deren pharmazeutisch annehmbare Salze oder Komplexe nach Anspruch 5, wobei die kognitiven Störungen durch degenerative Erkrankungen, wie Demenzen, Morbus Parkinson und dem Aufmerksamkeits-Defizit-Hypearaktivitäts-Syndrom (ADHS) bedingt sind.

7. Reserpin, Reserpinderivate und/oder deren pharmazeutisch annehmbare Salze oder Komplexe nach einem oder mehreren der Ansprüche 3 bis 6, **dadurch gekennzeichnet, daß** sie in einer Dosis von ca. 0,001 µg bis ca. 0,25 mg pro Tag, bevorzugt von ca. 0,002 mg - ca. 0,02 mg, besonders bevorzugt von ca. 0,01 bis ca. 0,06 µg, insbesondere von ca. 0,02 µg bis 0,04 µg pro Tag verabreicht, vorzugsweise oral verabreicht, werden.

8. Mittel enthaltend Reserpin, Reserpinderivate und/oder deren pharmazeutisch annehmbare Salze oder Komplexe zur Behandlung von Zuständen, die durch eine Hemmung des glutamatergen Systems bedingt sind.

9. Mittel nach Anspruch 8, wobei die Zustände durch eine Hemmung der metabotropen Glutamatrezeptoren, insbesondere der metabotropen Gruppe II-Glutamatrezeptoren bedingt sind.

10. Mittel enthaltend Reserpin, Reserpinderivate und/oder deren pharmazeutisch annehmbare Salze oder Komplexe zur Behandlung von kognitiven Störungen des zentralen Nervensystems, insbesondere bei degenerativen Erkrankungen, wie Demenzen, Morbus Parkinson und dem Aufmerksamkeits-Defizit-Hypearaktivitäts-Syndrom (ADHS).

11. Mittel nach einem der Ansprüche 8 - 10, enthaltend einen therapeutisch wirksamen Gehalt an dem Wirkstoff und optional übliche Hilfs- und Zusatzstoffe.

12. Mittel nach einem der Ansprüche 8 - 11, **dadurch gekennzeichnet, daß** sie den Wirkstoff in einer Dosis von ca. 0,001 µg bis ca. 0,25 mg pro Tag, bevorzugt von ca. 0,002 mg - ca. 0,02 mg, besonders bevorzugt von ca. 0,01 bis ca. 0,06 µg, insbesondere von ca. 0,02 µg bis 0,04 µg pro Tag enthalten.

13. Mittel nach einem der Ansprüche 8 - 12, **dadurch gekennzeichnet, daß** sie zur oralen Verabreichung hergerichtet sind.

14. Mittel nach einem oder mehreren der Ansprüche 8 - 13 zur Behandlung des Aufmerksamkeits-Defizit-Hyperaktivitäts-Syndroms (ADHS).
